# EUROPEAN PATENT APPLICATION

(11) **EP 3 995 136 A1**
(43) Date of publication of application: **11.05.2022**
(21) Application number: 20835303.7
(22) Date of filing: 13.05.2020
(51) Int. Cl.: A61K 9/28, A61K 9/50, A61K 31/18, A61P 13/00

(54) **PHARMACEUTICAL COMPOSITION CONTAINING TAMSULOSIN OR HYDROCHLORIDE THEREOF AND PREPARATION METHOD THEREFOR**

(30) Priority: 01.07.2019 KR 20190078517
(71) Applicant: Hanmi Pharm. Co., Ltd., Hwaseong-si, Gyeonggi-do 18536 (KR)
(72) Inventor: CHO, Hyuk Jun, Suwon-si, Gyeonggi-do 16358 (KR); KIM, Hwi, Suwon-si, Gyeonggi-do 16489 (KR); IM, Ho Taek, Yongin-si, Gyeonggi-do 16909 (KR); KIM, Yong Il, Gwacheon-si, Gyeonggi-do 13835 (KR)
(74) Representative: Schnappauf, Georg
(86) International application number: PCT/KR2020/006244
(87) International publication number: WO 2021/002588

(57) **Abstract**

Disclosed are a pharmaceutical composition containing tamsulosin or tamsulosin hydrochloride and a preparation method therefor. When forming an enteric coating layer, the content of talc is adjusted so that the enteric coating layer can be formed on core beads with the use of a fluid bed system, resulting in improvement in productivity. Even with a reduced content of talc, it is possible to minimize generation of aggregated enteric-coated beads, and the function and acid resistance of the enteric coating layer are not deteriorated after the core beads are coated. Therefore, the pharmaceutical composition is applicable to oral preparations.

## Description

### Technical Field

The present invention relates to a pharmaceutical composition containing tamsulosin or hydrochloride thereof and a preparation method therefor.

### Background Art

Tamsulosin hydrochloride is a drug that selectively acts on the genitourinary system by selectively inhibiting α-adrenoceptor. The tamsulosin hydrochloride is known to relax the smooth muscle surrounding the bladder and the prostate to improve the rate of urine excretion, to alleviate the symptoms of benign prostatic hyperplasia, and to exhibit significant efficacy with few side effects.

The bioavailability of tamsulosin hydrochloride is 90% or more so that it is well absorbed in the body. The half-life of tamsulosin is 9 to 13 hours in the bodies of healthy people while it is quite long (for example, 14 to 15 hours) in the bodies of benign prostatic hyperplasia patients. Accordingly, tamsulosin hydrochloride does not need to be provided as sustained extended-release preparations. When it is prepared to be slowly released for about 6 hours, the drug can be maintained at a sufficient concentration for 24 hours.

Tamsulosin hydrochloride is white crystalline powder and has physicochemical properties of being decomposed at about 230°C and of dissolving little in water. Since tamsulosin hydrochloride is not a substance that poorly dissolves in water, the release control thereof needs to be cautiously considered.

Tamsulosin hydrochloride formulations have an enteric coating layer formed by using a fluidized bed coater.

The coating process using a fluidized bed coater has the advantage that a coating solution can be uniformly coated on each particle (powder). However, the coating solution for the enteric coating layer has a problem in it is prepared to have a low viscosity for ease of spraying and thus the content of the solvent in the coating solution is inevitably high. As a result, the spraying time for forming the enteric coating layer to a predetermined thickness is prolonged, and the drying time for removing the solvent is also increased, thereby increasing the overall process time. When the production scale is large, this process time will inevitably be longer, resulting in lower productivity of the formulation.

To improve productivity, that is, to shorten the process time, the concentration and viscosity of the coating solution need to be increased by lowering the content of the solvent. However, in this case, a spray gun to spray the coating solution may be clogged due to the increased viscosity or beads may aggregate due to increase in adhesion, resulting variation in particle size.

Therefore, a new composition for the coating solution is required to improve productivity of formulations.

The inventors of the present application have made research on a method of improving the productivity of tamsulosin hydrochloride formulations while maintaining the function and acidic resistance of an enteric coating layer. As a result, when, as an anti-adhesion agent used for the formation of the enteric coating layer, a specific type of anti-adhesion agent is selected and the specific type of anti-adhesion agent is used in a specific content with respect to the total amount of the coating solution, it was found that it is possible to reduce the overall process time while securing the effect of inhibiting aggregation between particles during the coating process using a fluidized bed system.

### Disclosure

### Technical Problem

Objectives of the present disclosure are to provide a pharmaceutical composition containing tamsulosin or hydrochloride thereof and a preparation method therefor.

Other objectives of the present disclosure are to provide oral pharmaceutical preparations containing tamsulosin hydrochloride.

### Technical Solution

To achieve the above objectives, the present disclosure provides a tamsulosin-containing or tamsulosin hydrochloride-containing pharmaceutical composition and a preparation method therefor, the pharmaceutical composition including: one or more core beads containing tamsulosin or hydrochloride of tamsulosin as an active ingredient; and an enteric coating layer formed on each of the core beads.

In the pharmaceutical composition, talc may be contained in the enteric coating layer, in a ratio of 27% by weight or less, specifically in a ratio range of 7% to 25% by weight, with respect to a total solid content of the enteric coating layer.

The core bead may be an extended-release core bead coated with a release retarding layer including a release retarding agent.

The pharmaceutical composition may include a post mixture composition and the extended-release core bead coated with the enteric coating layer and may be formulated in the form of oral preparations.

### Advantageous Effects

According to the present disclosure, it is possible to reduce the content of a solvent in a coating solution for an enteric coating layer by adjusting a talc content, thereby shortening the process time for spraying and drying for enteric coating. Thus, the pharmaceutical composition according to the present disclosure can improve the productivity of formulations.

Although the pharmaceutical composition according to the present disclosure includes a reduced amount of talc, it is possible to minimize the production of post-agglomerated beads during the spraying process without deterioration of the function of the enteric coating layer and of the acid resistance. Therefore, the pharmaceutical composition according to the present disclosure can be applicable to oral preparations, particularly high-quality tablets.

### Description of Drawings

FIG. 1 is a graph showing change in productivity with a talc content for each of the tablets prepared according to Examples 1 to 4 and Comparison examples 1 and 2;

FIG. 2 is a graph showing change in a bead agglomeration ratio with a talc content in an enteric coating layer for each of the tablets prepared according to Examples 1 to 4 and Comparison examples 1 and 2; and

FIG. 3 is a graph showing change in dissolution rate with time for each of the tablets prepared according to Examples 1 to 4 and Comparison examples 1 and 2.

### Best Mode

Hereinafter, the present disclosure will be described in detail.

All technical terms used herein, unless otherwise defined, are used in the same sense as those understood by the ordinarily skilled in the related art. In addition, although preferred methods and samples are described herein, similar or equivalent ones fall within the scope of the present invention. The contents of all publications incorporated herein by reference are hereby incorporated by reference in their entirety.

In one aspect, a formulation including a tamsulosin- or tamsulosin hydrochloride-containing pharmaceutical composition has an enteric coating layer covering a core thereof containing tamsulosin or a hydrochloride form of tamsulosin for the purpose of oral administration.

A specific embodiment provides a tamsulosin- or tamsulosin hydrochloride-containing pharmaceutical composition including: one or more core beads containing tamsulosin or tamsulosin hydrochloride as an active ingredient; and
an enteric coating layer formed on each of the one or more core beads,
wherein the enteric coating layer may contain 27% by weight or less of talc with respect to a total solid content of the enteric coating layer.

The enteric coating layer can function to control the release of the active ingredient in the stomach with low pH such that the release rate of the active ingredient is at or below a specific level. In a specific embodiment, the enteric coating layer is formed through the steps of: preparing an enteric coating solution for the enteric coating layer; spraying the enteric coating solution in a manner of top spray, bottom spray, or tangential spray with the use of a fluidized bed system; and drying the sprayed enteric coating solution. When the production scale of formulations is increased, the time required for the spraying and drying processes is increased, resulting in a significant decrease in the productivity of the formulations. Accordingly, the present disclosure proposes a method capable of solving such a problem.

The enteric coating solution for the formation of the enteric coating layer may include one or more materials selected from among an enteric polymer, a plasticizer, an anti-adhesion agent, a solvent, and other additives. In a specific embodiment, the enteric coating solution may include an enteric polymer, a plasticizer, and an anti-adhesion agent. In another specific embodiment, the enteric coating solution may include an enteric polymer, a plasticizer, and talc.

Talc is used as the anti-adhesion agent to prevent adhesion between particles or adhesion of particles to the inner wall surface of the fluidized bed coater. As the anti-adhesion agent, aside from talc, magnesium stearate and glyceryl monostearate (GMS) may be used. The use of the magnesium stearate is limited due to interaction with an enteric polymer such as an anionic Eudragit used as an enteric polymer outer skin layer, and glyceryl monostearate requires a dissolution process using heated water having a temperature of 80°C before it is used. On the other hand, talc can be widely used because it is easily dispersed in solvents such as water and does not interact with other components, specifically for example, enteric polymer.

A typical enteric coating solution for forming an enteric coating layer uses about 50% by weight of talc relative to the solid content thereof. In order to improve productivity of formulations of a pharmaceutical composition, a redesign is required for the composition of the enteric coating solution.

The concentration of an enteric coating solution optimized for use in a fluidized bed coater ranges from 15 to 30% by weight, or from 17 to 28% by weight, or from 18 to 25% by weight, or preferably from 20±2% by weight. Herein, the concentration of the enteric coating solution refers to the total content of solid components in a coating solution including an enteric polymer, a plasticizer, talc, a solvent, and other additives as described above.

The concentration range is a range in which the coating solution can be uniformly coated on each bead having a core containing tamsulosin or a hydrochloride form of tamsulosin when it is coated with a fluidized bed coater. When the concentration is below the lower limit of the above concentration range, the coating time is prolonged and productivity is lowered. Conversely, when the concentration is beyond the upper limit of the above concentration range, the viscosity is excessively high so that a spray gun from which the coating solution is sprayed during the spraying process is clogged (gun block), or aggregation of the beads occurs due to the increase in adhesiveness, resulting in variation in the size of the produced beads. Therefore, the concentration of the enteric coating solution is preferably within the above range.

When designing the composition of the enteric coating solution, since the function or acid resistance of an enteric coating layer is affected by an enteric polymer and a plasticizer included in the enteric coating solution, it is advantageous to lower only the content of talc which is an inert component and does not affect the function of the enteric coating layer, with the content of each of the plasticizer and the enteric polymer fixed. As the content of talc is reduced, it is possible to reduce the amount of a solvent used for the coating solution. With the reduction in the solvent usage, the coating process time that is the sum of the spraying time and the drying time can be reduced.

Therefore, in the present disclosure, the content of talc in the enteric coating layer is limited to shorten the process time for forming the enteric coating layer, resulting in improvement in the productivity of formulation of the pharmaceutical composition with the advantage of maintaining the function and acid resistance of the enteric coating layer.

Specifically, talc is included in a ratio of 27% by weight or less, 25% by weight or less, 7% to 27% by weight, 7% to 25% by weight, 7% to 23% by weight, 8% to 22% by weight, or 9% to 22% by weight with respect to the total solid content of the enteric coating layer. The limited content range of talc is determined depending on the parameters of process time with the use of a fluidized bed coater, productivity, minimum content of aggregated particles and acid resistance.

When talc is present within the content range in the enteric coating layer, improvement in the productivity of formulation can be achieved through reduction in the production time, the formation of twin beads (aggregated beads) in which beads or tablets adhere to each other during the manufacturing process can be minimized, and the desired enteric coating function and acid resistance can be maintained.

When the content of talc in the enteric coating layer is high, there is an advantage that the number of aggregates of core beads is reduced due to the function of talc serving as an anti-adhesion agent but there is also a disadvantage that the amount of a solvent needs to be increased due to the increase in the solid content, resulting in increases in the overall process time including the spray process time and the drying process time.

On the contrary, when the content of talc is lowered to below the suggested range with the intention of reducing the overall process time, the process time can be shortened, but since the anti-adhesion effect of talc is insignificant, the adhesion of the core beads increases, causing agglomeration between the core beads. The enteric-coated core beads aggregated after the coating process are broken by the compression pressure applied thereto during a tableting process, resulting in damage to the enteric coating layer formed on the core beads. As a result, the acid resistance of tablets resulting from the tableting is greatly reduced, resulting in an increase in the dissolution rate of the tablets.

On the other hand, when talc usage is limited to the concentration range proposed in the present disclosure, the time required for the coating process is greatly reduced because the amount of the used solvent is reduced, resulting in improvement in productivity of formulation. According to Experimental Example 1, the process time was reduced from 310 minutes required in the case of a conventional composition to 239 minutes in the case of a composition according to the present disclosure, resulting in a productivity increase effect of about 22.9%. The time difference is only about 30 minutes, but when judging product productivity in an actual manufacturing process, the numerical value is very significant. Specifically, the larger the scale of manufacturing, the more significant the time difference is.

In addition, the limited content range of talc minimizes the aggregation between core beads by maintaining the anti-adhesion effect of talc. In the coating process of forming the enteric coating layer, aggregation between core beads is unavoidable however optimized the design of the composition is. The higher the content of the aggregated core beads, the lower the quality of tablets produced.

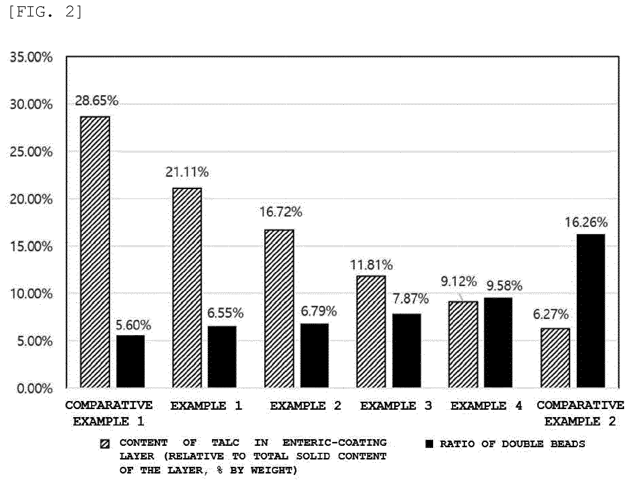

Conventionally, the ratio of aggregated core beads is about 5 to 6% by weight with respect to the total weight of the core beads. As the content of talc is lowered, the ratio of the aggregated beads increases. The ratio of aggregated core beads is about 10% by weight or less when talc is present within the content range presented in the present disclosure. As mentioned above, when the content of talc is reduced to an extremely low level, the process time can be shortened, but the ratio of aggregated beads increases to be a percentage of more than double digits (see FIG. 2).

Therefore, when talc usage is within the limited content range as presented in the present disclosure, the beneficial effects related to the process time for the formation of an enteric coating layer, productivity, and acid resistance can be obtained simultaneously.

Hereinafter, a pharmaceutical composition containing tamsulosin or a hydrochloride form thereof, according to the present disclosure, will be described in more detail.

Specifically, the pharmaceutical composition according to the present disclosure includes a core bead containing tamsulosin or tamsulosin hydrochloride and an enteric coating layer surrounding the surface of the core bead. The coated beads are mixed with a post mixture composition, and the final mixture is compressed. The core bead further includes a release retarding coating layer for controlled release.

### Core bead

The core bead is prepared to contain tamsulosin or tamsulosin hydrochloride as an active ingredient, or to contain a pharmaceutically acceptable inert seed as well as tamsulosin or tamsulosin hydrochloride as an active ingredient.

The tamsulosin or tamsulosin hydrochloride may be contained in an amount of 0.01 to 20% by weight, or 0.05 to 10% by weight, or 0.1 to 5% by weight relative to the amount of the total pharmaceutical composition. More specifically, the tamsulosin or tamsulosin hydrochloride may be contained in an amount of 0.1% to 1% by weight. The mentioned content range is the range of content with which sufficient efficacy can be expected after a dosage form (for example, tablet) of the pharmaceutical composition is being administered.

Although the core beads used in the present disclosure are not particularly limited in shape, size, and size variation, the core beads preferably have a spherical or near-spherical shape to allow for the enteric coating layer and the release retarding coating layer to be evenly formed over the entire surface of the core beads. To this end, the core beads are prepared through a process using a rotor system of GPCG-1, a wet extrusion process, a spherization process, or a granulation process.

To prepare the core beads, in addition to tamsulosin or tamsulosin hydrochloride that is an active ingredient, a pharmaceutically permissible binder may be further included.

The binder is a component that facilitates the binding between the components of the composition, thereby facilitating the shaping of the core beads. The binder used in the present disclosure is not particularly limited to a certain material, and any material that is commonly used in the related art may be used. Examples of the binder include hydroxy propyl methyl cellulose (HPMC), carboxy methyl cellulose, ethyl cellulose, methyl cellulose, hydroxy ethyl cellulose, ethyl hydroxy ethyl cellulose, hydroxypropyl cellulose (HPC), low-substituted HPC (L-HPC), polyvinyl pyrrolidone, polyvinyl alcohol, polymers of acrylic acid and acrylates, vinylpyrrolidone-vinylacetate copolymer (e.g. Kollidon^{®}), gelatin, guar gum, partially hydrolyzed starch, alginate, xanthan, and mixtures thereof. Specifically, hydroxy propyl methyl cellulose (HPMC) may be used.

In the pharmaceutical composition according to the present disclosure, the binder may be included in a content of 0.01% to 10% by weight, 0.05% to 5% by weight, and most preferably 0.1% to 1% by weight, relative to the total amount of the pharmaceutical composition. When the content of the binder is within that range, it is easy to prepare the core beads and to obtain desired dosage forms.

On the other hand, when preparing the core beads including an inert seed, the core beads are prepared in the form in which the active ingredient and the inert seed are mixed, the form in which the active ingredient is embedded in the inert seed, the form in which the active ingredient is coated on the surface of the inert seed, or any of other possible forms. In one embodiment of the present disclosure, the core bead is prepared in the form in which the active ingredient is coated on the surface of the inert seed.

An inert core is made from an inert material and serves as the core of the composition of the present disclosure. Specifically, the inert core may be made from one or more materials selected from sugar nuclei, starch, mannitol, sucrose, microcrystalline cellulose, and mixtures thereof. The size of the inert cores is preferably in a range of 100 to 1500 µm in terms of the productivity and yield of the fluidized bed assembling process. In the case of the form in which the active ingredient is embedded in the inert seed, the inert core may be implemented as a porous particle.

In the case of the form in which the active ingredient is coated on the surface of the inert seed, a coating solution including the active ingredient is prepared and the coating solution is applied to the surface of the inert seed by a coater, a fluidized bed system, a fluidized bed processor, a fluid bed granulator, or the like. More particularly, the coating may be carried out with a fluidized bed system, a centrifugal granulator, or Granurex^{®} (manufactured by Freund). Preferably, the coating may be carried out in a top spray manner, a bottom spray manner, or a tangential spray manner in a fluidized bed system.

### Release Retarding Coating Layer

The release retarding coating layer allows a medication to be slowly released to prevent side effects that may occur due to an immediate increase in the concentration of the medication in the blood when the medication is immediately released.

The release retarding coating layer is formed by using a release retarding agent (i.e., release modifying base). The release retarding agent not only serves to support a material forming a formulation but also plays an important role in forming pores in the formulation in water after a certain period of time. The release retarding agent used in the present disclosure is not specifically limited to a certain substance, and any material that is commonly used in the related art may be used.

The release retarding agent is one substance selected from the group consisting of cellulose derivatives, gums, hydrophilic or hydrophobic (meth)acrylate copolymers, hydrophilic or hydrophobic polyvinyl derivatives, polyethylene derivatives, carboxyvinyl compounds, and polysaccharides.

Specifically, the sustained release modifying agent includes at least one compound selected from the group consisting of hydroxypropylmethylcellulose, hydroxypropylcellulose, hydroxymethylcellulose, hydroxyethylcellulose, methylcellulose, ethylcellulose, carboxymethylcellulose sodium, cellulose acetate, cellulose acetate phthalate, methylhydroxyethylcellulose, guar gum, locust bean gum, tragacanth gum, carrageenan, acacia gum, xanthan gum, arabic gum, gellan gum, karaya gum, tara gum, tamarind gum, gati gum, polyvinyl alcohol, polyvinylpyrrolidone, polyvinyl acetyldiethylaminoacetate, polyethylene derivatives, polyethylene glycol, polyethylene oxide, carbomer, dextrin, polydextrin, dextran, pectin, pectin derivative, alginate, polygalacturonic acid, xylan, arabinoxylan, arabinogalactan, starch, hydroxypropyl starch, amylose, amylopectin, fluran, mannan, purcelleran, calageinan, glucosamine, chitosan, chitin, gelatin, collagen, casein, agar, albumin, poly(methacrylic acid-methyl methacrylate) copolymers, poly(methacrylic acid-ethyl methacrylate) copolymers, polyvinyl acetate, poly(ethyl acrylic acid-methyl methacrylate) copolymers, poly(ethyl acrylic acid-methyl methacrylate, and trimethylaminoethyl methacrylate) copolymers.

A specific example of the release retarding agent is at least one selected from among polyvinyl acetate, any mixture including polyvinyl acetate, ethyl cellulose, hydroxypropyl methylcellulose (HPMC), and any mixture thereof. When the release retarding agent is included, a certain release rate is exhibited regardless of pH. Therefore, according to the present disclosure, the desired release rate can be obtained.

According to the present disclosure, the most preferable release retarding agent is polyvinyl acetate or a certain mixture containing polyvinyl acetate. The polyvinyl acetate is most useful for making the formulation of the present disclosure have a sustained release property because it exhibits a constant release rate regardless of pH and allows for a sustained release over time when it is left underwater for a long period of time. The polyvinyl acetate may have a weight average molecular weight in a range of 100,000 to 500,000, but is not limited thereto. In the present disclosure, as the release retarding agent, polyvinyl acetate may be used solely or in combination with other substances and may be implemented as a diluted aqueous solution or a powder.

For example, the release retarding agent used in the present disclosure may be a mixture of polyvinyl acetate and polyvinyl pyrrolidone, or a mixture of polyvinyl pyrrolidone and polyvinyl acetate being stabilized with sodium laurylsulphate. Specifically, Kollidon^{®} SR (BASF, Germany) or Kollicoat^{®} SR30D^{®} (BASF, Germany) is used as the release retarding agent.

In the pharmaceutical composition of the present disclosure, the release retarding agent is included in a content of 0.1% to 15% by weight, in a content of 0.5% to 10% by weight, or in a content of 1% to 5% by weight, relative to the total weight of the pharmaceutical composition. When the content of the release retarding agent is below the lower limits of the range, the effect expected for the release retarding agent cannot be obtained. On the other hand, when the content of the release retarding agent exceeds the upper limit of the range, the release rate of a medication cannot be easily controlled. Therefore, the content of the release retarding agent in the pharmaceutical composition must be suitably controlled within the range described above.

The release retarding coating layer is formed by: preparing a coating solution composed of the release retarding agent and a solvent selected from among purified water, ethanol, acetone, ethanol aqueous solution, acetone aqueous solution, and a mixture of ethanol and acetone; and spraying the coating solution using a coater, a fluidized bed system, a fluidized bed processor, or a fluidized bed granulator. More specifically, a fluidized system with bottom spray, a centrifugal granulator, or GRANUREX^{®} (manufactured by Freund corporation) is be used.

In addition to the composition described above, the core beads used in the present disclosure additionally contains a variety of biologically inactive ingredients for various supplemental purposes such as coating efficiency, stability of active ingredients, appearance, color, protection, maintenance, binding, performance improvement, manufacturing process improvement, or auxiliary release control, etc.

The biologically inactive ingredients may be added when preparing the cores and will have little or no effect on the release of the active ingredients. Examples of the additional inactive ingredients include diluents, sugars, sugar alcohols, polymers, colorants, flavoring agents, sweeteners, surfactants, lubricants, stabilizers, antioxidants, foaming agents, paraffin, and waxes. One or more additives selected from the examples may be added. The biologically inactive ingredient may be mixed with at least one of the tamsulosin, the hydrochloride form of tamsulosin, the inert seed, and the release retarding agent.

For example, the biologically inactive ingredient may be a diluent.

The diluent refers to a material that allows granules to remain in their shape and do not melt in the body. As the diluent, one or more materials selected from among microcrystalline cellulose (MCC), talc, lactose, and inorganic carriers such as dibasic calcium phosphate, dibasic calcium phosphate dihydrate, tribasic calcium phosphate, and combinations thereof may be used. In a specific embodiment, at least one may be selected from examples such as among microcrystalline cellulose and talc but the examples will not be limited thereto.

The core beads are made from a mixture of the active ingredient, the inert seed, and the release retarding agent and are prepared in various forms.

The core beads used in one embodiment of the present disclosure are spherical core beads and are made from a mixture of tamsulosin or tamsulosin hydrochloride, a binder, and inert seeds.

According to another embodiment of the present disclosure, as the core beads, extended-release core beads are used which are prepared by primarily coating inert seeds with a primary coating solution containing tamsulosin or tamsulosin hydrochloride and a binder and then secondarily coating the surface of the primarily coated inert seeds with a secondary coating solution containing the release retarding agent.

According to a further embodiment of the present disclosure, as the core beads, extended-release core beads are used which are prepared by coating inert seeds with a primary coating solution containing tamsulosin or tamsulosin hydrochloride, a binder, and a lubricant, and then coating the surface of the primary coated inert seeds with a secondary coating solution containing the release retarding agent and the lubricant.

In addition to the above-described forms of the core beads, selection of specific materials for forming the core beads, the use of the specific materials, and a method of adding the specific materials to the core beads of the present disclosure may be easily varied without departing from the scope of the present disclosure when considering the skill level of those ordinarily skilled in the art.

### Enteric Coating Layer

The enteric coating layer according to the present disclosure refers to an enteric coating material(enteric polymer) layer covering the surface of a core bead. The enteric coating layer functions to control the release of tamsulosin or tamsulosin hydrochloride in the stomach with low pH such that the release rate is at or below a specific level. This prevents the side effects of tamsulosin or tamsulosin hydrochloride on the gastrointestinal tract and facilitates the release of tamsulosin hydrochloride in the intestine, thereby improving the bioavailability of tamsulosin or tamsulosin hydrochloride.

The enteric coating layer preferably contains an enteric polymer. The enteric polymer is a pH-dependent polymer. The enteric polymer is stable in an acidic condition, which is below pH 5, but dissolves in a weak acidic condition, which is at or above pH 5 or above, or in a neutral condition.

The enteric polymer used in the present disclosure is not limited to a specific polymer, but any enteric polymer that is commonly used in the related art can be used. The enteric polymer may be selected from among hydroxypropylmethylcellulose phthalate, hydroxypropylmethylcellulose, acetate succinate, polyvinyl acetate phthalate, cellulose acetate phthalate, cellulose acetate trimellitate, shellac, poly(meth)acrylate, polymethyl(meth)acrylate, poly(meth)ethyl acrylate, polyethyl acrylate, poly (meth)methyl acrylate, copolymers thereof, and blended forms thereof.

In a specific embodiment, the enteric polymer may include at least one acid-resistant acrylic polymer selected from among methacrylic acid-ethyl acrylate copolymer, methacrylic acid-ethyl methacrylate copolymer, and ethyl acrylate-methyl methacrylate copolymer.

In a specific embodiment, the enteric polymer may include at least one of methacrylic acid-ethyl acrylate copolymer and ethyl acrylate-methyl methacrylate copolymer.

Preferably, an acid-resistant acrylic polymer is used as the enteric polymer. The acid-resistant acrylic polymer is pH-dependent so that it does not dissolve in strong acidic conditions (about pH 4.5 or lower) but dissolves at pH 5.5 or higher. The acid-resistant acrylic polymer used in the present disclosure may be selected from the group consisting of Eudragit^{®} L30 D-55, Eudragit^{®} NE30D, Eudragit^{®} L100, Eudragit^{®} L100 D-55, Eudragit^{®} L12,5, Eudragit^{®} S100, Eudragit^{®} S12,5, Eudragit^{®} FS 30 D, and any combination thereof.

In a specific embodiment, as the enteric polymer, a mixture of Eudragit^{®}L30 D-55 (methacrylic acid-ethyl acrylate copolymer) and Eudragit^{®} NE30D (ethyl acrylate-methyl methacrylate copolymer dispersion) mixed in a weight ratio in a range of from 0.5:1 to 1:0.5, and preferably in a weight ratio of 1:1.

Eudragit^{®} L30 D-55 is a methacrylic acid-ethylacrylate copolymer (1:1) aqueous dispersion. It poorly dissolves in a low pH (for example, pH 5.5 or lower) condition but begins to dissolve when the pH becomes 5.5 or higher. Due to this characteristic, it is widely used as an enteric polymer. The Eudragit^{®} NE30D is an ethyl acrylate-methyl methacrylate (1:1) copolymer and is available as a 30% (m/V) dispersion.

The enteric polymer is contained in a ratio of 55% to 90% by weight, 60% to 90% by weight, 65% to 90% by weight, 70% to 85% by weight, or 70% to 83% by weight with respect to the solid content of the enteric coating layer. When the content of the enteric polymer is within any one of the ranges, the function of the entering coated layer can be achieved. That is, the release of a drug within the gastrointestinal tract can be suppressed. When the content of the enteric polymer is below the lower limit of the range, drugs may release in the gastrointestinal tract due to the low acid resistance. Conversely, when the content of the enteric polymer is higher than the upper limit of the range, it is difficult to prepare the desired dosage form (for example, tablet) because the viscosity of the pharmaceutical composition is increased and is thus difficult to be coated.

In other words, the content of the enteric polymer may be in a range of 1% to 40% by weight, 2% to 35% by weight, 3% to 30% by weight, or 5% to 20% by weight, relative to the total weight of the pharmaceutical composition.

The enteric polymer can increase the acid resistance of dosage forms, but when the pharmaceutical composition is formulated in the form of tablets, damage to the enteric coating layer made of the enteric polymer may occur during the tableting process. Therefore, the acid resistance of the finished dosage form (i.e., tablet) is lowered. For this reason, after being administered, the active ingredient may release from the tablet in the gastrointestinal tract after, resulting in significant deterioration of the bioavailability of the drug.

Accordingly, a plasticizer is used to increase the acid resistance of the pharmaceutical composition by inhibiting damage to the enteric coating layer during the tableting process.

The plasticizer refers to a material that increases the free volume of a polymer to facilitate segmental motion of the polymer. The plasticizer serves to increase durability of a dosage form of drug by imparting flexibility and elasticity to the coating layer. There are various kinds of plasticizers such as aromatic plasticizers and aliphatic plasticizers. Among them, a suitable one may be selected taking into account biocompatibility, miscibility, stability, processability, persistence, and price, etc.

Typically as the plasticizer, one or more materials selected from the group consisting of triethyl citrate, dibutyl phthalate, diethyl phthalate, dibutyl sebacate, diethyl sebacate, tributyl citrate, acetyl triethyl citrate, acetyl triethyl citrate, propylene glycol, triacetin, polyethylene glycol, cetyl alcohol, stearyl alcohol, and cetostearyl alcohol may be used. Preferably, triethyl citrate is used as the plasticizer.

The plasticizer is contained in a ratio of 3% to 20% by weight, 4% to 18% by weight, 5% to 16% by weight, or 6% to 15% by weight with respect to the solid content of the enteric coating layer. When the plasticizer is included in an amount that falls within any one of the ranges described above, the enteric coating layer has sufficient acid resistance and stability. When the content of the plasticizer is below those ranges, the acid resistance of the enteric coating layer is reduced, resulting in high initial and final release rates of the drug in the stomach. Conversely, when the content of the plasticizer exceeds those ranges, there is a problem in that tamsulosin or tamsulosin hydrochloride is decomposed. Therefore, the content of the plasticizer needs to be suitably determined within any of the ranges described above.

In other words, the content of the plasticizer may be in a range of from 0.1% to 20% by weight, 0.15% to 15% by weight, 0.1% to 10% by weight, 0.2% to 5% by weight relative to the total weight of the pharmaceutical composition.

Talc is used as an anti-adhesion agent and is used together with the enteric polymer and the plasticizer. As already mentioned above, the content of talc may be within a range that is determined depending on various parameters such as process time required when a fluidized bed system is used, productivity, agglomerated bead ratio, and acid resistance.

In addition to the components described above, as a solvent for the preparation of the enteric coating solution, purified water, ethanol, acetone, ethanol aqueous solution, acetone aqueous solution, or a mixture of ethanol and acetone may be used. The content of the solvent may be determined such that the concentration of the enteric coating solution can be adjusted to be within a range of from 15% to 30% by weight, from 17% to 28% by weight, from 18% to 25% by weight, or preferably from 18 to 22% by weight.

In addition, in addition to the components described above, various biologically inactive additives may be added to the composition of the enteric coating solution for auxiliary purposes such as efficiency of coating, stability of the active ingredient, appearance, color, protection, retention, binding, improvement in performance, improvement in manufacturing process or auxiliary release control, etc.

The additives can be diluents, sugars, sugar alcohols, polymers, colorants, flavoring agents, sweeteners, surfactants, lubricants, stabilizers, antioxidants, foaming agents, preservatives, disintegrants, buffering agents, bulking agents, diluents, paraffin, or wax, and at least one of these may be used as the additives. The ratio of the total additives with respect to the solid content of the entire coating layer may be 5% by weight or less. In this case, even when the additives are not used, it is preferable that the ratio of talc is not be changed from the range given above.

### Oral Preparation

The pharmaceutical composition according to the present disclosure may be formulated in the form of oral preparations.

The oral preparations can be used to cure any diseases known as indications for tamsulosin or its hydrochloride salt, as well as for the treatment of any diseases that may be discovered as indications in the future. In the present disclosure, the term "cure" has meaning including treatment, improvement, amelioration, and management of a disease. In a specific embodiment, the pharmaceutical preparations may be used to cure benign prostatic hyperplasia, urination disorders accompanying prostatic hyperplasia, and acute urinary retention.

An oral preparation according to the present disclosure may contain 0.2 to 0.8 mg of tamsulosin or tamsulosin hydrochloride per unit dosage form thereof. Even though the oral preparation is administered once a day, the active ingredient may exhibit an optimized dissolution pattern.

In a specific embodiment, the tamsulosin- or tamsulosin hydrochloride-containing pharmaceutical composition may be formulated in the form of a composite preparation including additional active ingredients that can be used in combination with tamsulosin or tamsulosin hydrochloride, as well as tamsulosin or tamsulosin hydrochloride. The additional active ingredients may include, for example, tadalafil, dutasteride, solifenacin, mirabegron or finasteride, but may not be limited thereto.

Oral preparations include powders, granules, pellets, tablets, capsules, dry syrup formulations, syrup formulations, and jelly formulations, etc. Among these, the pharmaceutical composition according to the present disclosure may be formulated as tablets. The tablets include general tablets commonly used in the field of the present disclosure, double tablets, chewable tablets, and fast disintegrating tablets.

As an oral preparation, tablets have the advantages of providing an accurate dosage, being easily handled, being easily taken, being easily mass-produced, and being inexpensive. In addition, it is easy to correct bitter taste, odor, and irritation through the coating, and it is easy to control the dissolution and absorption patterns of the drug by controlling the shaping.

For example, tablets can be manufactured through a tableting process. In this case, core beads coated with an enteric coating layer, a post mixture composition, and additives are mixed, and then a certain level of tableting pressure is applied to the final mixture to produce tablets. As a result, the core beads coated with the enteric coating layer and the post mixture composition are in a compressed state after being mixed.

The tableting is a process of binding particles by generating a binding force between the particles with the use of a tablet press. Specific steps are not particularly limited in the present disclosure.

A tableting machine is an apparatus for molding powder or granules under strong pressure with punches and dies of various shapes, and can produce tablets of about 50 mg to about 1500 mg depending on the size and shape of the punch and die.

Tablets may vary in shape. For example, the tables may take an ovoid, triangular, almond, peanut, parallelogram, circular, pentagonal, hexagonal, or trapezoidal shape. The desirable table shape is round, ovoid, or parallelogram.

The post mixture composition may be a composition used for commonly used for tablet production. The post mixture composition used in the present disclosure includes at least one of a disintegrant and a buffering agent. The disintegrant and the buffering agent are components required for the preparation of tablets. The composition thereof is determined in consideration of the process characteristics of the oral preparation prepared through tableting.

The disintegrant refers to a composition that can maintain an appropriate hardness of an oral preparation and can easily decompose an oral preparation to be easily absorbed in the body when the oral preparation is administered. As the disintegrant, one material or two or more materials may be selected from those commonly used for the production of tablets. For example, mannitol (e.g., spray-dried mannitol), sorbitol, fructose, lactose, dextrose, xylitol, glucose, lactose, white sugar, fructose, maltose, maltodextrin, erythritol, arabitol, crospovidone, croscarmellose sodium, microcrystalline cellulose (MCC), pregelatinzed starch, starch, derivatives of starch, Kollidone^{®}, carboxymethyl cellulose, F-Melt^{™} (manufactured by Fuji chemical), Ludiflash^{®} (manufactured by BASF), and the like can be used as the disintegrant. Specifically, the disintegrant includes at least one material selected from among mannitol, F-Melt^{™}, sorbitol, xylitol, lactose, and pregelatinized starch. More specifically, the disintegrant is mannitol, F-Melt, sorbitol, xylitol, or mixtures thereof. The spray-dried mannitol may be prepared by spraying and drying a crystalline mannitol solution, or by purchasing a commercially available form (for example, PEARLITOL SD 200WP manufactured by Roquette in France, or PARTECK M200 manufactured by Merck in Germany)

The content of the disintegrant in the post mixture composition is 50% to 90% by weight relative to the total amount of the pharmaceutical composition. When the content of the disintegrant is below the range described above, the oral preparation is easily decomposed during transport, preservation, or storage. In this case, product defects occur or rapid dissolution occurs after the drug is taken. Therefore, drug efficacy cannot be expected due to fast release of the medication. Conversely, when the content of the disintegrant is excessively high, excessively fast dissolution occurs, resulting in a high initial release rate of the active ingredient. As a result, it is difficult to obtain a long-lasting effect.

The post mixture composition used in the present disclosure may further include a buffering agent. In a specific embodiment, colloidal silica (AEROSIL^{®}) is used as the buffering agent. In addition to the colloidal silica, other buffering agents also can be used.

The buffering agent can not only lower the bulk density but also prevent abrasion, friction, and breakage of the core bead, which are related to the quality of the oral preparations, during the tableting process, thereby suppressing the dissolution of the active ingredient in the gastrointestinal tract. That is, the buffering agent increases the acid resistance. In addition, the use of the buffering agent increases the fluidity and dispersion of particles during the tableting process, thereby allowing the enteric-coated core beads to be uniformly contained in each tablet.

Specifically, the content of the buffering agent is set to be in a range of from 0.5% to 3.0% by weight and preferably in a range of from 0.5% to 2.5% by weight with respect to the amount of the pharmaceutical composition. When the content is determined to be within the range, high content uniformity and acid resistance can be obtained. When the content of the buffering agent is below the range, the effects described above cannot be achieved. Conversely, when the content exceeds the range, the bulk density will decrease, the friability will increase, and the content uniformity and acid resistance of the oral preparation will decrease.

In addition to the composition, the oral preparation of the present disclosure additionally contains a variety of biologically inactive ingredients for various supplemental purposes such as coating efficiency, stability of the active ingredient, appearance, color, protection, maintenance, binding, performance improvement, manufacturing process improvement, or auxiliary release control, etc.

The inactive ingredient may be a diluent, sugar, sugar alcohol, polymer, colorant, flavoring agent, sweetener, surfactant, lubricant, stabilizer, antioxidant, foaming agent, preservative, disintegrant, buffering agent, bulking agent, diluent, paraffin, or wax. Among them, one or more inactive ingredients may be used. For example, the biologically inactive ingredient may be a lubricant.

The lubricant exhibits glidant or antitacking effects so that tablets can be easily released from a compression die, problems such as adhesion can be eliminated during tableting, and possibility of cracking or delamination of tablets can be prevented during tableting. As the lubricant, one or more materials selected from the group consisting of stearic acid, glyceryl behenate, glyceryl mono stearate, magnesium stearate, calcium stearate, sodium stearyl fumarate, silicon dioxide, talc, and magnesium silicate. Preferably, magnesium stearate is used as the lubricant.

The content of the lubricant is 10% by weight or less, 0.1% to 10% by weight, or 0.5% to 5% by weight, with respect to the total pharmaceutical composition. The use of the lubricant offers the advantage of preventing sticking during tableting, thereby lowering the defect rate caused by problems occurring in tablet manufacturing. However, the excessive use of the lubricant makes it difficult to satisfy the dissolution rate standard of medications. Therefore, the content of the lubricant is preferably determined within the specified range.

The oral preparation having the composition according to the present disclosure has a dissolution rate of 20% or less after 2 hours when the dissolution rate is measured with a pH 1.2 buffering agent of 500 mL and a rotational speed of 75 rpm according to the paddle method, which is the second dissolution test according to the United States Pharmacopoeia (USP). Therefore, it is possible to minimize dissolution in the stomach and maximize dissolution in the bowel.

In addition, the oral preparation according to the present disclosure satisfies a uniformity level of 15 that is a standard of uniformity of dosage unit for a tamsulosin hydrochloride tablet, which is specified in the United States Pharmacopoeia (USP).

The present disclosure provides a method for preparing the pharmaceutical composition described above.

In another aspect, the present disclosure provides a method of preparing a pharmaceutical composition containing tamsulosin or tamsulosin hydrochloride, the method including: preparing one or more core beads containing tamsulosin or tamsulosin hydrochloride as an active ingredient; and

forming an enteric coating layer on the surface of the core bead using an enteric coating solution.

The enteric coating solution contains 27% by weight or less of talc with respect to the total solid content of the enteric coating solution. The preparation method will be described, step by step, in more detail below.

### Mode for Invention

Hereinbelow, the embodiments of the present disclosure will be described in more detail with reference to examples. However, the examples described below are provided only to aid understanding of the present disclosure and thus should not be construed as limiting to the scope of the present invention.

### Preparation Example 1: Preparation of Extended-Release Core Bead

Core beads containing **tamsulosin hydrochloride** and coated with a release retarding coating layer were prepared.

First, tamsulosin hydrochloride, hydroxypropylmethylcellulose (HPMC) serving as a binder, ethanol, and purified water were mixed in a content ratio shown in Table 1 in a high speed mixer, and then talc was added to the mixture to produce a first coating solution. Microcrystalline cellulose (MCC, Cellets^{®} 175, with a particle size of 150 to 200 µm) as inert seeds was put into a fluidized bed system, and the first coating solution was bottom-sprayed to the inert seeds. After the spraying of the first coating solution was finished, the resultant seeds were dried to produce core beads containing tamsulosin hydrochloride.

To form a release retarding coating layer on the surface of each of the core bead, Kollicoat^{®} SR 30D(EP), which is a release retarding agent, was dissolved in water, and talc was added thereto to produce a release retarding coating solution. The prepared core beads were put into a fluidized bed system, and the coating was carried by bottom-spraying the release retarding coating solution to the prepared core beads. After the spraying of the release retarding coating solution was finished, the resultant beads were dried to produce extended-release core beads with the release retarding coating layer thereon.

**[Table 1]**

| | Component | mg/T |
|---|---|---|
| Core bead | Microcrystalline cellulose (Cellet^{®} 175) | 20.00 |
| | Tamsulosin hydrochloride | 0.40 |
| | Hydroxypropyl methylcellulose | 0.20 |
| | Talc | 0.20 |
| | Ethanol | (25.20) |
| | Purified water | (2.80) |
| Total for core beads | | 20.80 |
| Release retarding coating solution | Kollicoat^{®} SR 30D | 4.49 |
| | Talc | 0.46 |
| | Purified water | (14.96) |
| Total for extended-release core beads | | 25.75 |

### Experimental Example 1: Comparison of Physical Properties between Tablets that Differ in Content of Talc

Tablets include an enteric coating layer that is formed on the extended-release core beads using a fluidized bed spraying process. In this case, the differences in the process time and the quality of tablets occur depending on the content of talc used as an anti-adhesion agent for the enteric coating layer. In the present experimental example, tablets were prepared with various enteric coating solutions that were varied in the content of talc, and the physical properties of the prepared tablets were compared.

### <Preparation of Tablets>

Tablets were prepared in accordance with the compositions shown in Table 2. As core beads, the extended-release core beads prepared in Preparation Example 1 were used.

To prepare the enteric coating solutions, Eudragit^{®} L30-D55 (methacrylic acid-ethyl acrylate 1:1 copolymer contained in a concentration of 30%) and Eudragit^{®} NE30D (ethyl acrylate-methyl methacrylate copolymer contained in a concentration of 30%) were used as enteric coating materials, and triethyl citrate was used as a plasticizer. The Eudragit^{®} L30-D55 and the Eudragit^{®} NE30D were dissolved in purified water in a mixer, and talc and triethyl citrate were added thereto to prepare the enteric coating solutions. In each enteric coating solution, each of the Eudragit^{®} L30-D55 and Eudragit^{®} NE30D, which are dispersions, was used by 11.32 mg as shown in Table 2 below, and the content of solid components in each of the dispersions was 3.4 mg.

In Examples 1 to 4, the content of talc within the content of solid components in each enteric coating solution varied within the range of from 0.75 mg to 2 mg, whereas in Comparative Examples 1 and 2, the content of talc was 3 mg and 0.5 mg, respectively.

The extended-release core beads prepared in Preparation Example 1 were collected and put into a fluidized bed system, and the enteric coating was carried out by spraying each of the prepared entering coating solutions from the bottom. After the spraying of the enteric coating solution was finished, microspheres (i.e., enteric-coated extended-release core beads) obtained by forming an enteric coating layer on each of the extended-release core beads were obtained.

A post mixture composition including a microcrystalline cellulose-mannitol composite mixture (disintegrant), colloidal silicon dioxide (buffering agent), and magnesium stearate (lubricant) was added to the obtained enteric-coated core beads, followed by tableting to prepare tablets. The content in Table 2 below is a weight ratio with respect to the total weight of solid components.

**[Table 2]**

| Ingredients (mg/T) | | Exampl e 1 | Exampl e 2 | Exampl e 3 | Exampl e 4 | Comparati ve Example 1 | Comparati ve Example 2 |
|---|---|---|---|---|---|---|---|
| Tamsulosin hydrochloride-containing extended-release core beads | | 25.75 | 25.75 | 25.75 | 25.75 | 25.75 | 25.75 |
| Enteri c coatin g layer | Methacrylic acid-ethyl acrylate copolymer | 3.4 | 3.4 | 3.4 | 3.4 | 3.4 | 3.4 |
| | Ethyl acrylate-methyl methacrylate copolymer | 3.4 | 3.4 | 3.4 | 3.4 | 3.4 | 3.4 |
| | Triethyl citrate | 0.67 | 0.67 | 0.67 | 0.67 | 0.67 | 0.67 |
| | Talc | 2 | 1.5 | 1 | 0.75 | 3 | 0.5 |
| | Purified water | (18.7) | (16.8) | (14.8) | (13.8) | (22.64) | (12.8) |
| Content of talc in enteric coating layer (% by weight relative to the total solid content of the layer) | | 21.11 | 16.72 | 11.81 | 9.12 | 28.65 | 6.27 |
| Enteric-coated core | | 35.22 | 34.72 | 34.22 | 33.97 | 36.22 | 33.72 |
| beads | | | | | | | |
| Post mixtur e | Microcrystall ine cellulose-mannitol composite mixture | 158.78 | 159.28 | 159.78 | 160.03 | 157.78 | 160.28 |
| | Colloidal silicon dioxide | 4 | 4 | 4 | 4 | 4 | 4 |
| | Magnesium stearate | 2 | 2 | 2 | 2 | 2 | 2 |
| Grand Total (mg) | | 200 | 200 | 200 | 200 | 200 | 200 |

### <Evaluation of Productivity>

Productivity for tableting is related to the time of each process step, meaning that the longer the process time for the step of forming the enteric coating layer, the lower the productivity. This relates to the composition of the enteric coating solution for forming the enteric coating layer. Productivity for each of the compositions of the examples and the comparative examples was evaluated by measuring the process time for forming the enteric coating layer using each of the compositions prepared in the examples and the comparative examples. The process time means the sum of the time required for a spraying step in the case of using a fluidized bed system and the time required for a drying step. In this case, the shorter the time, the higher the productivity.

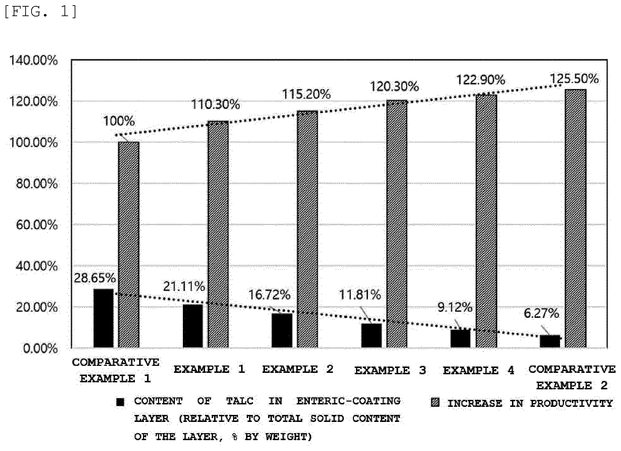

In Comparative Example 1, the content of talc is a ratio of talc used in a conventional process. Relative productivity for each of the other examples and comparative examples was measured with respect to the process time of Comparative Example 1. The evaluation results obtained are shown in Table 3 and FIG. 1. The concentration of each enteric coating solution was in the range of 21±0.5% by weight.

**[Table 3]**

| | Comparative Example 1 | Example 1 | Example 2 | Example 3 | Example 4 | Comparative Example 2 |
|---|---|---|---|---|---|---|
| Content of talc in enteric coating layer (% by weight relative to the total solid content of the layer) | 28.65 | 21.11 | 16.72 | 11.81 | 9.12 | 6.27 |
| Total weight of components of coating solution | 38.49 mg | 34.55 mg | 32.65 mg | 30.65 mg | 29.65 mg | 28.65 mg |
| Process time | 310 min | 278 min | 263 min | 247 min | 239 min | 231 min |
| Increase in productivity | 100% | 110.30% | 115.20% | 120.30% | 122.90% | 125.50% |
| (relative value) | | | | | | |

Referring to Table 3, it can be seen that as the talc content in the enteric coating layer decreases with the concentration of the coating solution fixed, the process time is shortened and the productivity is improved.

FIG. 1 is a graph showing the productivity improvement according to the talc content in the tablets prepared in Examples 1 to 4 and Comparative Examples 1 and 2. The graph indicates that the productivity linearly increases in inverse proportional to the increase in the talc content in the enteric coating layer.

The process time was 310 minutes in Comparative Example 1 and the process time was maximally reduced to 239 minutes in Example 4. That is, Example 4 shows an increase of about 22.9% in productivity. The time difference is only about 30 minutes, but this numerical value is very significant when evaluating the productivity of manufacture of products in an actual manufacturing process. Specifically, the larger the scale of manufacturing, the more significant the reduced time is.

### <Evaluation of Production Rate of Agglomerated Beads>

It is ideal that the enteric coating layer is uniformly formed on the core beads. However, when the content of talc, which is an anti-adhesion agent, in the enteric coating layer is low, aggregation between the core beads occurs after the coating process. Such agglomeration is unavoidable in actual tablet production, and thus it is desirable to minimize the agglomeration of core beads.

The ratio of agglomerated core beads was determined by sieving the core beads with a 60-mesh (i.e., 250 µm) sieve and measuring the amount of coarse particles remaining on the sieve. The remaining particles on the sieve were regarded as aggregated beads, and the ratio of the amount of the remaining particles with respect to the total amount of the sieved beads was obtained.

In Comparative Example 1, the content of talc is a talc content used in a conventional process. The ratio of aggregated core beads obtained in Comparative Example 1 was set as a reference value, and the ratio for each of the other comparative examples and the examples was compared against the reference value. The results obtained are shown in Table 4 and FIG. 2.

**[Table 4]**

| | Comparative Example 1 | Example 1 | Example 2 | Example 3 | Example 4 | Comparative Example 2 |
|---|---|---|---|---|---|---|
| Content of talc in enteric coating layer (% by weight relative to | 28.65 | 21.11 | 16.72 | 11.81 | 9.12 | 6.27 |
| the total solid content of the layer) | | | | | | |
| Enteric-coated core beads | 1.054 g | 1.008 g | 1.104 g | 1.016 g | 1.086 g | 1.033 g |
| Remaining particles | 0.059 g | 0.066 g | 0.075 g | 0.080 g | 0.104 g | 0.168 g |
| Ratio of aggregated beads | 5.60% | 6.55% | 6.79% | 7.87% | 9.58% | 16.26% |

Referring to Table 4, in the case of the enteric-coated core beads of Comparative Example 1, about 94% or more passed through the sieve and the remaining amount in the sieve was about 5.6%. As the content of talc in the enteric coating layer decreased, the ratio of the remaining beads in the sieve increased. Comparative Example 2 shows a prominent increase in the ratio of the aggregated beads.

FIG. 2 is a graph showing change in the ratio of aggregated beads with change in the talc content in the enteric coating layer. Referring to FIG. 2, the ratio of aggregated beads in Examples 1 to 4 only slightly increased while the talc content linearly decreased in Comparative Examples 1 and 2 and Examples 1 to 4. However, it was confirmed that the ratio of aggregated beads in Comparative Example 2 was three times or more higher than that in Comparative Example 1. An increase in the ratio of the agglomerated beads poses the problem of an increase in dissolution rate in the acid resistance test described below.

### <Acid Resistance Test>

The productivity evaluation result shows that Comparative Example 2 exhibits an increase of about 25.5% in productivity compared to Comparative Example 1. That is, it seems advantageous that the talc content is small. However, the low talc content brings a disadvantageous result in terms of acid resistance. This can be confirmed through the present test.

To evaluate the acid resistance of each tablet prepared in Examples 1 to 4 and Comparative Examples 1 and 2, a dissolution rate according to time was observed.

### - Dissolution test conditions

1) Dissolution method: US Pharmacopoeia (USP) dissolution test method 2 (paddle method)
2) Dissolution solution: Artificial gastric fluid 500 ml (pH 1.2)
3) Volume of dissolution solution: 500 ml
4) Temperature of dissolution instrument: 37±0.5°C
5) Paddle speed: 75 rpm
6) Number of test groups: 6
7) Sample Collection and Analysis Method

In 30, 60, and 120 minutes after the start of the test, 10 ml of each dissolution test solution was collected, filtered, and subjected to high performance liquid chromatography (HPLC method) under analysis conditions described below.
- Analysis conditions
- Columns: 4.6 x 150 mm, 5 µm, ODS or similar columns
- Mobile phase: perchlorinated aqueous solution (adjusted to pH 2.0 with sodium hydroxide):acetonitrile = 5:2
- Detector: Ultraviolet absorption spectrophotometer (wavelength: 225 nm)
- Temperature: about 40°C
- Flow rate: 1.0 mL/min
- Filling volume: 400 µl

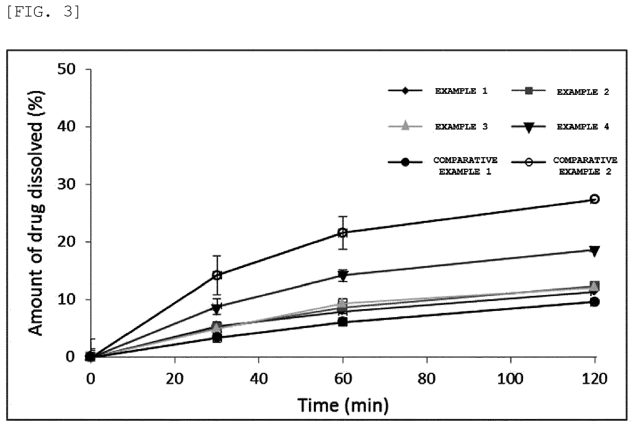

The results obtained are shown in Table 5 and FIG. 3, wherein the numbers in parentheses represent the deviations. FIG. 3 is a graph showing change in dissolution rate with time for each of the tables prepared according to Examples 1 to 4 and Comparison Examples 1 and 2.

The lower the dissolution rate, the better the acid resistance. It is preferable that the dissolution rate after 2 hours is 20% or less. A higher number of the deviations in the parentheses represents less uniformity among dosage units.

**[Table 5]**

| | Dissolution rate over time (%)(deviation) | | | |
|---|---|---|---|---|
| | 0 | 30 minutes | 60 minutes | 120 minutes |
| Example 1 | - | 5.3(0.7) | 8.0(0.8) | 11.4(0.4) |
| Example 2 | - | 5.1(0.8) | 8.7(0.9) | 12.3(0.9) |
| Example 3 | - | 4.9(1.2) | 9.3(0.8) | 12.1(0.8) |
| Example 4 | - | 8.8(1.4) | 14.2(1.3) | 18.6(1.0) |
| Comparative Example 1 | - | 3.4(0.8) | 6.1(0.8) | 9.6(0.7) |
| Comparative Example 2 | - | 14.2(3.1) | 21.6(3.4) | 27.4(2.9) |

Referring to Table 5 and FIG. 3, as the content of talc increased, the dissolution rate decreased, showing a tendency that the acid resistance improved.

The tablets of Examples 1 to 4 showed a dissolution rate of 20% or less after 2 hours, indicating good acid resistance.

On the contrary, in the case of the tablet of Comparative Example 1, the dissolution rate was low because the content of talc was higher than that of each of the tablets of Examples 1 to 4. However, Comparative Example 1 had the problem that the productivity was low productivity as confirmed through the productivity evaluation.

In addition, in the case of the tablet of Comparative Example 2, it exhibited a relatively high dissolution rate indicating relatively poor acid resistance compared to the tablets of Examples 1 to 4 due to the relatively low talc content. In the case of the tablet of Comparative Example 2, the ratio of agglomerated beads after the enteric coating was significantly increased as confirmed through the evaluation of generation of agglomerated beads. The aggregated beads were broken during tableting and the enteric coating layer was damaged, resulting in a high dissolution rate.

Therefore, it can be seen that for the control of the talc content, the acid resistance of tablets associated with the dissolution rate must be considered as well as the productivity improvement effect attributable to the reduced drying time.

### <Content Uniformity Test and Test Result>

To evaluate the content uniformity of tamsulosin hydrochloride throughout each tablet, it was checked whether the uniformity of dosage unit for each tamsulosin hydrochloride tablet satisfies a reference level of 15 or lower which is specified in the United States Pharmacopoeia (USP).

10 tablets prepared in each of the examples and comparative examples were collected and tested. Each tablet was put in a 50 mL flask, 10 mL of methanol was added thereto, and ultrasound extraction was performed for 30 minutes. After cooling, the marks of the flasks were aligned with a mobile phase, and the solutions of the flasks were filtered with 0.45 µm-mesh or finer membrane filters. The filtrates were taken as samples, and the samples were subjected to high-performance liquid chromatography (HPLC). The HPLC conditions were as follows:

### - Analysis conditions

Columns: 4.6 x 150 mm, 5 µm, ODS or similar columns
Mobile phase: perchlorinated aqueous solution (adjusted to pH 2.0 with sodium hydroxide):acetonitrile (5:2) Detector: ultraviolet absorption spectrophotometer (wavelength: 225 nm)
Temperature: about 40°C
Flow rate: 1.0 mL/min
Filling volume: 50 µl

In the content uniformity test, the smaller the value, the more uniformly the content of tamsulosin hydrochloride is present in each tablet.

**[Table 6]**

| | Exampl e 1 | Exampl e 2 | Exampl e 3 | Exampl e 4 | Comparativ e Example 1 | Comparativ e Example 2 |
|---|---|---|---|---|---|---|
| Determinatio n value | 2.1 | 2.6 | 3.1 | 2.3 | 2.9 | 7.4 |
| Standard | 15 or less | | | | | |

As a result of the content uniformity test, it was confirmed that all of the tablets according to Examples 1 to 4 and Comparative Examples 1 and 2 exhibited very good content uniformity. That is, the values indicating the uniformity level were less than 15 that was set a reference level defined in the US Pharmacopoeia.

## Claims

1. A tamsulosin- or tamsulosin hydrochloride-containing pharmaceutical composition comprising: one or more core beads comprising tamsulosin or tamsulosin hydrochloride as an active ingredient; and an enteric coating layer formed on each of the core beads, wherein the enteric coating layer contains 27% by weight or less of talc with respect to a total solid content of the enteric coating layer.

2. The pharmaceutical composition of claim 1, wherein the talc is contained in a content of 7% to 25% by weight with respect to the total solid content of the enteric coating layer.

3. The pharmaceutical composition of claim 1, wherein the enteric coating layer contains an enteric polymer and a plasticizer.

4. The pharmaceutical composition of claim 3, wherein the enteric polymer comprises at least one acid-resistant acrylic polymer selected from methacrylic acid-ethyl acrylate copolymers, methacrylic acid-ethyl methacrylate copolymers, and ethyl acrylate-methyl methacrylate copolymers.

5. The pharmaceutical composition of claim 3, wherein the plasticizer comprises at least one material selected from the group consisting of triethyl citrate, dibutyl phthalate, diethyl phthalate, dibutyl sebacate, diethyl sebacate, tributyl citrate, acetyl triethyl citrate, acetyl triethyl citrate, propylene glycol, triacetin, polyethylene glycol, cetyl alcohol, stearyl alcohol, and cetostearyl alcohol.

6. The pharmaceutical composition of claim 1, wherein the core bead comprises a release retarding coating layer containing a release retarding agent, the release retarding coating layer being formed on a surface of the core bead.

7. The pharmaceutical composition of claim 1, further comprising: a disintegrant, a buffering agent, or both.

8. The pharmaceutical composition of claim 1, wherein the pharmaceutical composition exhibits a dissolution rate of 20% or lower after 2 hours when the dissolution rate is measured with a pH 1.2 buffer of 500 mL at a rotation speed of 75 rpm according to the USP dissolution test method 2 (paddle method).

9. A pharmaceutical preparation produced using the pharmaceutical composition of any one of claims 1 to 8.

10. The pharmaceutical preparation of claim 9, wherein the pharmaceutical preparation is in the form selected from the group consisting of powders, granules, pellets, tablets, capsules, dry syrup formulations, syrup formulations, or jelly formulations.

11. The pharmaceutical preparation of claim 10, wherein the pharmaceutical preparation is in the form of tablets.

12. A method of preparing a tamsulosin- or tamsulosin hydrochloride-containing pharmaceutical composition, the method comprising: preparing one or more core beads comprising tamsulosin or tamsulosin hydrochloride as an active ingredient; and forming an enteric coating layer by coating the core beads with an enteric coating solution, wherein the enteric coating solution contains 27% by weight or less of talc with respect to a solid content of the enteric coating solution.

13. The method of claim 12, wherein the talc is contained in a content of 7% to 25% by weight with respect to the solid content of the enteric coating solution.

14. The method of claim 12, wherein the enteric coating solution comprises an enteric polymer in a content of 55% to 90% by weight with respect to the solid content of the enteric coating solution.

15. The method of claim 12, wherein the enteric coating solution comprises a plasticizer in a content of 3% to 20% by weight with respect to the solid content of the enteric coating solution.

16. The method of claim 12, wherein the enteric coating solution has a concentration in a range of 15% to 30% by weight.

17. The method of claim 12, wherein the forming of the enteric coating layer is performed using a fluidized bed system.
